# EUROPÄISCHE PATENTSCHRIFT

(11) **EP 2 931 952 B1**
(45) Veröffentlichungstag und Bekanntmachung des Hinweises auf die Patenterteilung: **30.01.2019**
(21) Anmeldenummer: 13814460.5
(22) Anmeldetag: 04.12.2013
(51) Int. Cl.: D01F 2/10

(54) **REGENERIERTE CELLULOSEFASER**
REGENERATED CELLULOSE FIBRE
FIBRE DE CELLULOSE RÉGÉNÉRÉE

(30) Priorität: 13.12.2012 EP 12197065
(43) Veröffentlichungstag der Anmeldung: 21.10.2015
(73) Patentinhaber: Kelheim Fibres GmbH, 93309 Kelheim (DE)
(72) Erfinder: ROGGENSTEIN, Walter, 93077 Bad Abbach (DE); HERMANUTZ, Frank, 71229 Leonberg (DE); WIMMER, Philipp, 93047 Regensburg (DE)
(74) Vertreter: Nemec, Harald
(86) Internationale Anmeldenummer: PCT/EP2013/075580
(87) Internationale Veröffentlichungsnummer: WO 2014/090665

(56) Entgegenhaltungen:
- CH-A- 359 832
- DE-A1-102007 054 702
- DE-B- 1 222 204
- US-A- 5 008 385

## Beschreibung

Die vorliegende Erfindung betrifft eine nach dem Viskoseverfahren erhaltene regenerierte Cellulosefaser.

Insbesondere beschäftigt sich die vorliegende Erfindung mit einer regenerierten Cellulosefaser mit hydrophoben Eigenschaften.

Die Verwendung von Alkylketendimeren (AKD) und weiteren Fettsäurederivaten als Leimungsmittel zur Hydrophobierung von Papier ist bekannt (z.B. DE 3 920 356). Hierbei wird die Cellulose im Papier durch den Fettsäurerest der Reaktanden, welcher über eine Esterbindung angebunden wird, hydrophobiert. Einsatzbereiche sind beispielsweise Schreibpapiere, Photopapiere und Lebensmittelverpackungen.

Ebenso ist die Beeinflussung der prozentualen Wasseraufnahme von Viskosefasern durch Inkorporierung hydrophiler und / oder hydrophober Hilfsstoffe seit längerer Zeit bekannt. Die Hilfsstoffe tragen zur Gesamtmasse der Fasern bei, nehmen jedoch selbst kein Wasser auf. Beispiele sind: Bariumsulfat, Schwermetallsulfide, Kohlenstoff, Polyolefine (DE 1 469 448 A1, DE 3 317 724 A1, US 2 234 734 A).

Bei einer solchen Einbringung von Füllstoffen wird im Regelfall ausschließlich ein Masseneffekt generiert, und zwar bedingt durch die Einrechnung des Füllstoffanteils in die Gesamtmasse. Die Gesamtwasseraufnahme der Faser wird zwar hierbei herabgesetzt, weil die Füllstoffe selbst kein Wasser aufnehmen, zumeist jedoch keine hydrophobe Wirkung erzielt. Das Füllen der Faser führt zudem zu einem erheblichen Verlust an Faserfestigkeit.

Die DE 3 317 724 A1 erwähnt zusätzlich die Inkorporation von hydrophoben, oligomeren oder polymeren Substanzen, wobei konkret inerte Polymere wie Polyethylen, Polypropylen, PTFE etc. genannt werden.

Ebenfalls bekannt ist eine Nachbehandlung von Fasern mit Nanopartikeln unter Verwendung von Bindemitteln (DE 10 2006 053326 A1), durch Aufpfropfung hydrophober Stoffe auf Cellulose (z.B. Methylmethacrylat: DE 1 468 539 A), durch Hydrophobierung mittels Reaktion cellulosischer Textilfasern mit Polyiso- bzw. Polyisothiocyanat (GB 586549A), durch reaktive Nachbehandlung von Faserstoffen / Textilien, z.B. Veretherung über Acetalbindung (GB 477 029A), Veresterung mit Säurechloriden in Organischen Lösungsmitteln wie Pyridin und DMF (FR 707 688A bzw. Textile Research Journal, 40 (1970), 970 ff.).

Es wurde auch beschrieben, Fettsäurechloride in einem inerten Lösungsmittel einzubringen und unreagierte Reaktanden sowie Chlorwasserstoff als Reaktionsprodukt im Heißgasstrom weiter- und auszutragen (DE 69801056T2, WO 2012/066015 A, FR 2 767 270 A1).

Die alkoholatinduzierte Veresterung von Fasern mit Fettsäuresalzen (GB 780967A) wurde ebenso beschrieben wie die textile Nachbehandlung mit AKD-Dispersionen (GB 2 221 928A).

Diese vorbekannten Nachbehandlungen werden zum einen als Beschichtung durchgeführt, also lediglich auf der Oberfläche der Faser bzw. des textilen Artikels. Zum anderen können diese reaktiv durchgeführt werden, wobei allerdings auch hier die Reaktion nur in oberflächennahen Bereichen der Faser stattfindet, da die Reaktanden nicht ungehindert ins Faserinnere vordringen können. Außerdem ist der Verbrauch an Hydrophobierungsmittel hoch, da beispielsweise im Falle der Nachbehandlung mit AKD dieses bereits in der Hitze mit dem im deutlichen Überschuß vorhandenen Wasser abreagiert und somit nicht mehr für eine Reaktion mit der Cellulose zur Verfügung steht.

Durch die Nachbehandlung wird eine wasserabweisende Oberfläche ausgebildet, nach deren Zerstörung die Faser allerdings ungehindert und irreversibel Wasser aufsaugt.

Die gasphasenunterstützte Hydrophobierung von Oberflächen wurde mit Hilfe von Papiermustern demonstriert, an denen jedoch deutliche Unterschiede zwischen den beiden Seiten des Papieres erkennbar waren. Die hydrophobe Wirkung ließ sich durch Extraktion mit Aceton größtenteils entfernen, was auf einen hohen Anteil an Hydrolyse bei der Reaktion der Fettsäurechloride mit der Cellulose hindeutete. Auch in diesem Fall können die Fettsäurechloride in der Gasphase nicht frei ins Innere der Faser diffundieren sondern binden primär außen an.

Weiters ist die sogenannte "Animalisierung" von Spinnfasern durch Einspinnung eines Polymerisationsproduktes aus aromatischem Iso- bzw. Isothiocyanat und cyclischem Imin bzw. einem Polymerisat des letzteren bekannt (CH 213876). Dabei wurde eine teilweise Hydrophobierung beobachtet, dies allerdings nur bei sehr hohen Additivierungen mit einem wasserabweisenden polymeren Wirkstoff, dessen Verteilung ausreichte, um die Oberflächenenergie der Faser ausreichend herabzusetzen. Die so erhaltenen Fasern weisen zudem aufgrund des hohen Addivgehaltes eher den Charakter von Wollfasern auf.

Zur Verbesserung der Entformbarkeit von Wurst aus Viskose-Wursthüllen wurden diese mit AKD in wässriger Suspension nachbehandelt, oder aber die Viskosemasse vor der Formung der Hülle mit AKD-Suspension oder Emulsion versetzt (GB 887 466A, GB 1 042 182 A). Eine wasserabweisende Wirkung wird in diesen Dokumenten nicht beschrieben, erwähnt wird lediglich die höhere Affinität der Hülle zu Fetten. Der grundlegende Unterschied zwischen Viskosefasern und Wursthüllen besteht darin, dass die Wursthüllen im Regelfall aus mit Viskosemasse getränkten Papieren bestehen, in seltenen Fällen aus reiner Viskose. In letzterem Fall liegt die Viskose nicht in Faserform, sondern als dicker Schlauch mit Wandstärken von ca. 50 µm und mehr vor.

Die vorliegende Erfindung stellt sich zur Aufgabe, eine nach dem Viskoseverfahren erhaltene regenerierte Cellulosefaser mit hydrophoben Eigenschaften zur Verfügung zu stellen, welche die oben geschilderten Nachteile der Vorschläge des Standes der Technik nicht aufweist. Die Faser sollte zudem biologisch abbaubar sein.

Diese Aufgabe wird mit einer regenerierten Cellulosefaser gelöst, welche eine hydrophobe Substanz, ausgewählt aus der Gruppe bestehend aus Alkylketendimeren, Alkenylketendimeren, Alkylsuccinanhydriden, Alkenylsuccinanhydriden, Alkylglutarsäureanhydriden, Alkenylglutarsäureanhydriden, Alkylisocyanaten, Alkenylisocyanaten, Fettsäureanhydriden sowie Mischungen daraus in der Cellulosematrix inkorporiert aufweist.

Es wurde überraschenderweise gefunden, dass die Inkorporation reaktiver hydrophober Substanzen wie AKD in die Cellulosematrix einer regenerierten Cellulosefaser erfolgreich durchgeführt werden kann. Dadurch wird über den gesamten Faserquerschnitt verteilt eine hydrophobierende Wirkung erzielt, die "permanent" ist, d.h. durch oberflächliche Behandlung der Fasern wie z.B. Waschschritte oder andere Behandlungen nicht entfernt wird.

Es wurde weiters gefunden, dass durch die Inkorporation reaktiver hydrophober Substanzen in die Cellulosematrix die grundlegenden Eigenschaften der Viskosefasern, z.B. die Möglichkeit Wasserdampf aufzunehmen, nicht beeinträchtigt werden.

Zur Inkorporation der hydrophoben Substanzen müssen diese der Spinnviskose oder einem Vorläufer davon zugegeben werden. Es ist dabei überraschend, dass Substanzen wie AKD sich im Viskosespinnverfahren als stabil erwiesen haben, da sich z.B. AKD im Alkalischen schnell zersetzt.

In der erfindungsgemäßen Cellulosefaser beträgt der Gehalt an hydrophober Substanz in der Faser bevorzugt 0,1 Gew.% bis 13 Gew.%, besonders bevorzugt 1 Gew.% bis 7,5 Gew.%, bezogen auf Cellulose.

Der Titer der erfindungsgemäßen Faser kann 0,5 bis 40 dtex, insbesondere 2 bis 28 dtex betragen.

Die erfindungsgemäße Faser kann als Kurzschnittfaser mit einer Schnittlänge von 2 bis 20 mm, besonders bevorzugt 3 bis 12 mm vorliegen. Insbesondere für die Anwendung in Vliesstoffen und Textilien kann die Faser auch als Stapelfaser in Schnittlängen von 20 mm bis 150 mm, insbesondere 40 bis 110 mm, besonders bevorzugt 40 mm (Baumwolltype) und 70 mm (Wolltype) vorliegen.

Das Verfahren zur Herstellung einer erfindungsgemäßen Cellulosefaser umfasst den Schritt der Zugabe der hydrophoben Substanz zu einer Spinnviskose oder einem Vorläufer davon.

Unter "Spinnviskose" versteht der Fachmann eine wässerige, alkalische Lösung von Cellulosexanthogenat. Als "Vorläufer" einer Spinnviskose sind Einsatzstoffe und Zwischenprodukte des Viskoseverfahrens zu verstehen, z.B. der eingesetzte Zellstoff, der Zellstoff nach Alkalisierung, oder auch die zur Auflösung des Cellulosexanthogenates eingesetzte Löselauge.

Die vorliegende Erfindung betrifft auch die Verwendung der erfindungsgemäßen Cellulosefaser in Hygieneprodukten, insbesondere in Coversheets/Backsheets, in Kosmetik- und Babywischtüchern, in Medizinprodukten, insbesondere in Wundauflagen, in Papieren und Nassvliesen, in textilen Anwendungen, insbesondere Sportbekleidung und Schutzbekleidung und/oder in Vliesstoffen und Filtermedien, insbesondere Zigarettenfiltern.

### Beispiele

In eine Spinnviskose wurden hydrophobe Substanzen der folgenden Substanzklassen zugegeben:
a) Ein Vinylacetat-Copolymer (Vinnapas EN 1028, Hersteller Fa. Wacker)
b) Ein quaternäres Fettsäurederivat (Adulcinol BUN, Hersteller Fa. Zschimmer & Schwarz)
c) Ein Alkylketendimer (AKD) (Ukasol NL-201, Hersteller Fa. Schill & Seilacher)
d) Ein weiteres quaternäres Fettsäurederivat (Stantex s6557, Hersteller Fa. Pulcra Chemicals)
e) Eine Fettalkohol-Kombination (Setilon KN, Hersteller Fa. Pulcra Chemicals)
f) Ein Fettsäurekondensationsprodukt (Duron OS 2160, Hersteller Fa. CHT) und
g) Esteröle (Duron OS 3136, Hersteller Fa. CHT)

Es wurde versucht, aus den solcherart modifizierten Spinnviskosen auf übliche Art und Weise Viskosefasern zu verspinnen. Eine erfolgreiche Ausspinnung von Fasern war nur in den Fällen c) und g) möglich.

Eine Erhöhung der Hydrophobie der hergestellten Fasern (untersucht anhand des Einsinkens der Fasern in Wasser) konnte nur im Fall c) (Hydrophobierung mit AKD) beobachtet werden.

## Patentansprüche

1. Regenerierte Cellulosefaser erhalten nach dem Viskoseverfahren, welche eine hydrophobe Substanz ausgewählt aus der Gruppe bestehend aus Alkylketendimeren, Alkenylketendimeren, Alkylsuccinanhydriden, Alkenylsuccinanhydriden, Alkylglutarsäureanhydriden, Alkenylglutarsäureanhydriden, Alkylisocyanaten, Alkenylisocyanaten, Fettsäureanhydriden sowie Mischungen daraus in der Cellulosematrix inkorporiert aufweist.

2. Cellulosefaser gemäß Anspruch 1, **dadurch gekennzeichnet, dass** der Gehalt an hydrophober Substanz in der Faser 0,1 Gew.% bis 13 Gew.%, vorzugsweise 1 Gew.% bis 7,5 Gew.%, bezogen auf Cellulose, beträgt.

3. Verfahren zur Herstellung einer Cellulosefaser gemäß einem der vorhergehenden Ansprüche, umfassend den Schritt der Zugabe der hydrophoben Substanz zu einer Spinnviskose oder einem Vorläufer davon.

4. Verwendung einer Cellulosefaser gemäß einem der Ansprüche 1 bis 3 in Hygieneprodukten, insbesondere in Coversheets/Backsheets, in Medizinprodukten, insbesondere in Wundauflagen, in Papieren und/oder Nassvliesen, in textilen Anwendungen, insbesondere Sportbekleidung und Schutzbekleidung und/oder in Vliesstoffen und Filtermedien, insbesondere Zigarettenfiltern.

## Claims

1. A regenerated cellulose fibre obtained by the viscose process, which contains a hydrophobic substance selected from the group consisting of alkyl ketene dimers, alkenyl ketene dimers, alkyl succinic anhydrides, alkenyl succinic anhydrides, alkyl glutaric acid anhydrides, alkenyl glutaric acid anhydrides, alkyl isocyanates, alkenyl isocyanates, fatty acid anhydrides as well as mixtures thereof incorporated in the cellulose matrix.

2. A cellulose fibre according to claim 1, **characterized in that** the content of the hydrophobic substance in the fibre ranges from 0.1% by weight to 13% by weight, preferably from 1% by weight to 7.5% by weight, based on cellulose.

3. A process for the production of a cellulose fibre according to any of the preceding claims, comprising the step of adding the hydrophobic substance to a spinning viscose or a precursor thereof.

4. The use of a cellulose fibre according to any of claims 1 to 3 in sanitary products, in particular in cover sheets/back sheets, in medical products, in particular in wound dressings, in papers and/or wet-laid nonwovens, in textile applications, in particular sportswear and protective clothing and/or in nonwoven fabrics and filter media, in particular cigarette filters.

## Revendications

1. Fibre de cellulose régénérée obtenue selon le procédé viscose présentant une substance hydrophobe choisie dans le groupe constitué de dimères d'alkylcétène, de dimères d'alcényl-cétène, d'anhydrides succiniques d'alkyle, d'anhydrides succiniques d'alcényle, d'anhydrides de l'acide alkyl-glutarique, d'anhydrides de l'acide alcényl-glutarique d'isocyanates d'alkyle, d'isocyanates d'alcényle, d'anhydrides d'acide gras ainsi que des mélanges de ces substances, incorporée dans la matrice cellulose.

2. Fibre de cellulose selon la revendication 1, **caractérisée en ce que** la teneur en substance hydrophobe dans la fibre est de 0,1 à 13 % en poids, de préférence de 1 à 7,5 % en poids, par rapport à la cellulose.

3. Procédé de fabrication d'une fibre de cellulose selon l'une quelconque des revendications précédentes, comprenant l'étape de l'ajout de la substance hydrophobe à une viscose discontinue ou à un précurseur.

4. Utilisation d'une fibre de cellulose selon l'une quelconque des revendications 1 à 3 dans des produits d'hygiène, en particulier des feuilles de couverture/des feuilles supports, dans des produits médicaux, en particulier dans des pansements, dans des papiers et/ou des non-tissés obtenus par voie humide, dans des applications textiles, en particulier les vêtements de sport et les vêtements de protection et/ou dans des tissus non-tissés et des supports filtrants, en particulier les filtres de cigarette.
